# EUROPEAN PATENT SPECIFICATION

(11) **EP 3 189 855 B1**
(45) Date of publication and mention of the grant of the patent: **21.03.2018**
(21) Application number: 16386001.8
(22) Date of filing: 11.01.2016
(51) Int. Cl.: A61K 31/661, A61K 47/10, A61K 47/26, A61K 9/08

(54) **ORAL PHARMACEUTICAL SOLUTIONS CONTAINING HYDROCORTISONE SODIUM PHOSPHATE**
ORALE PHARMAZEUTISCHE LÖSUNGEN MIT HYDROCORTISON-NATRIUM-PHOSPHAT
SOLUTIONS PHARMACEUTIQUES ORALES DE PHOSPHATE DE SODIUM CONTENANT DE L'HYDROCORTISONE

(43) Date of publication of application: 12.07.2017
(73) Proprietor: Lamda Laboratories S.A., Attiki (GR)
(72) Inventor: Karatzas, Angelos, 15238 Chalandri, Attiki (GR); Stappa, Argyro, 15231 Chalandri, Attiki (GR); Apostolou, Konstantinos, 15344 Gerakas, Attiki (GR); Loukas, Agathoklis, 15343 Agia Paraskevi (GR); Resvani, Amalia, 11851 Petralona (GR)
(74) Representative: Roukounas, Dimitrios

(56) References cited:
- US-A- 2 970 944
- US-A- 3 238 102
- US-A- 3 696 195
- US-A1- 2005 186 229

## Description

### TECHNICAL FIELD

The present invention relates to pharmaceutical solutions suitable for oral administration comprising hydrocortisone sodium phosphate.

### BACKGROUND OF THE INVENTION

Hydrocortisone (Chemical formula I), is chemically named 11β,17α,21-Trihydroxypregn-4-ene-3,20-dione or 4-Pregnene-11b,17a,21-triol-3,20-dione

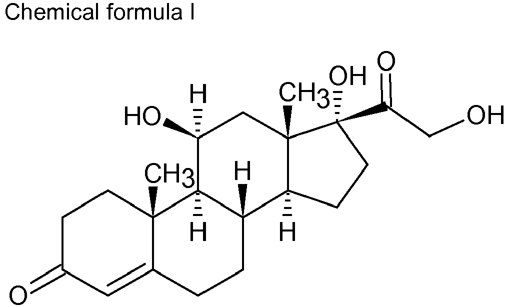

Hydrocortisone belongs to a group of anti-inflammatory medicines known as corticosteroids. The mechanism of action of hydrocortisone is as a Corticosteroid Hormone Receptor Agonist. Hydrocortisone is the name given to natural cortisol hormone, produced by the adrenal glands, when it is used as a medication. As a glucocorticoid receptor agonist, hydrocortisone promotes protein catabolism, gluconeogenesis, capillary wall stability, renal excretion of calcium, and suppresses immune and inflammatory responses.

Since hydrocortisone is practically insoluble in water, the water-soluble sodium phosphate salt of hydrocortisone (Chemical formula II) may be used instead as an active ingredient for the preparation of liquid pharmaceutical solutions, thus ensuring good water solubility.

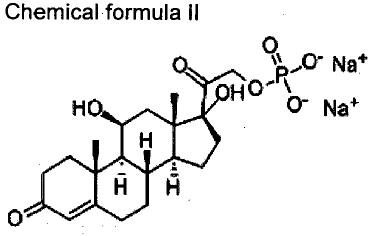

Although a large number of solutions for injection of hydrocortisone, hydrocortisone sodium succinate and hydrocortisone acetate are commercially available in several countries, no pharmaceutical solutions suitable for oral administration comprising hydrocortisone sodium phosphate are currently commercially available.

U.S. Pat. No. 2,970,944 discloses that although aqueous solutions of steroid phosphate salts are colourless and free from insoluble matter when freshly made, these solutions, begin to deteriorate upon standing at room temperature or at elevated temperatures. The deterioration is evidenced by the formation of a precipitate and by the development of a yellow colour. This condition may be due to hydrolysis of the ester, whereby the free alcohol is precipitated or to other factors such as oxidative degradation. Solutions stabilized with creatinine are disclosed such as the formulation described in example 3 that contains 20 mg/ml hydrocortisone phosphate (as monosodium salt), creatinine, sodium bisulfite, phenol, sodium hydroxide q.s. pH 7.5 and Water for injection.

U.S. Pat. No. 2,786,835 discloses that various ingredients may be useful in solutions containing hydrocortisone or its derivatives, including stabilizers such as sodium bisulfite or sodium formaldehyde sulfoxylate.

The commercially available sterile aqueous solution "Hydrocortisone 13.39% w/v solution for injection", which is marketed in the UK by Amdipharm Mercury Company Limited, contains 133.9 mg/ml hydrocortisone sodium phosphate equivalent to 100 mg/ml hydrocortisone. This product also comprises, as excipients, disodium edetate, disodium hydrogen phosphate, anhydrous sodium acid phosphate, phosphoric acid (10% solution), water for injections and sodium formaldehyde bisulphite monohydrate as stabilizer. The product is packaged in 1 ml and 5 ml neutral glass ampoules.

According to the British Pharmacopoeia the pH of injection solutions that contain hydrocortisone sodium phosphate as active ingredient is from 7.0 to 8.0. According to the Summary of Product Characteristics the product should be stored at a temperature below 25°C.

The sterile aqueous solution Hydrocortone® Phosphate Injection that is commercially available in the United States of America is a solution (pH 7.5 to 8.5), sealed under nitrogen, for intravenous, intramuscular, and subcutaneous administration. Each ml of solution contains hydrocortisone sodium phosphate equivalent to 50 mg hydrocortisone. This solution also comprises as excipients 8 mg creatinine, 10 mg sodium citrate, sodium hydroxide to adjust pH, and water for injection, q.s. 1 ml, with 3.2 mg sodium bisulfite, 1.5 mg methylparaben, and 0.2 mg propylparaben added as preservatives. This product is packaged in 2 ml single dose vials.

Sodium bisulfite is mainly a food additive. On July 8, 1986, sodium bisulfite was banned from use by the FDA on fresh fruits and vegetables in the United States following the deaths of 13 people and many illnesses, mainly among asthmatics.

Sodium metabisulfite is used as a disinfectant, antioxidant and preservative agent. It is used as a preservative and antioxidant in food. It may cause allergic reactions in those who are sensitive to sulfites, including respiratory reactions in asthmatics, anaphylaxis and other allergic reactions in sensitive individuals. Thus, such excipients are unsuitable for pharmaceutical compositions intended to treat asthmaticus and acute allergic reactions, such as compositions comprising hydrocortisone sodium phosphate.

Sodium formaldehyde bisulfite (or sodium hydroxymethanesulfonate), under alkaline conditions is believed to slowly hydrolyze into formaldehyde and sodium bisulfite which in turn provide the desired antibacterial and antioxidant preservative properties, respectively. However, U.S. Pat. No. 4,402,943 and U.S. Pat. No. 4,555,522 disclose that certain sulfites, bisulfites and related compounds, such as sodium formaldehyde bisulfite, display antithrombotic activity, involving anti-coagulant and/or platelet antiaggregatory activities, as well as antihypertensive activity.

As apparent from the review of the prior art documents and the commercially available pharmaceutical solutions, all disclosed compositions comprising hydrocortisone sodium phosphate are intended for packaging in single dose containers. Furthermore, none of the proposed compositions can be used in the form of pharmaceutical solutions for oral administration since they comprise antioxidants and/or stabilizing agents which are not recommended to be used in higher concentrations in order to physicochemically stabilize and effectively preserve drug products intended for packaging in multi-dose containers.

A significant challenge arises when oral solutions prone to atmospheric oxidation are provided in multi-dose containers because of the risk of microbial contamination, or physicochemical degradation of the solution, once the closure system has been breached, due to repeated opening and closing after first use by the patient.

For parenteral drugs exclusion of oxygen from a formulation can be achieved for example by packaging the drug in glass ampoules that are heat sealed under an inert atmosphere. However, it is very difficult to formulate oxygen-sensitive drugs such as hydrocortisone sodium phosphate in multi-dose vials, which are sealed with a polymer stopper. Polymers are reasonably permeable to oxygen. Thus, oxidatively unstable drugs require more than just oxygen exclusion during sealing to prevent oxidation from occurring.

Technically the biggest challenge is the development of effectively preserved formulations. The most important problem is the use of excipients with low toxicity in the lowest feasible concentrations without compromising the stability of the formulations. In other words, the low concentrations of preservatives contribute in reducing the potential for toxicological effects, but low concentrations may be insufficient for achieving the required level of antimicrobial preservation.

The challenge of effectively preserving an oral pharmaceutical solution known to be more stable at pH values of around 7.5 or higher becomes more complex by the fact that sulfites, bisulfites and related compounds can potentially cause adverse reactions or undesirable pharmacological effects, as shown above, while other commonly used preservatives are either not effective in basic solutions or they are not chemically stable. For example most acidic preservatives are not effective above their pKa. That is if the pH is higher than the pKa, more of the acid will be in the ionized form, thus potentially rendering the preservative ineffective. Additionally, although the group of alkyl esters of p-hydroxybenzoic acid (parabens) has an effective pH range of 4.0 to 8.0, it is well known to undergo hydrolysis in weak alkaline solutions i.e. above pH 7.0.

The present invention addresses the problems of the prior art knowledge by advantageously providing pharmaceutical solutions of hydrocortisone sodium phosphate suitable for oral administration, which exhibit excellent shelf life after first opening (in-use shelf life).

### SUMMARY OF THE INVENTION

The present invention provides aqueous pharmaceutical solutions of hydrocortisone sodium phosphate suitable for oral administration which exhibit excellent shelf life after first use.

According to the invention, the oral pharmaceutical solutions comprise hydrocortisone sodium phosphate as active ingredient, 100 mg/ml to 200 mg/ml propylene glycol, 100 mg/ml to 200 mg/ml sorbitol, a pharmaceutically acceptable buffering agent and purified water wherein the pH of the oral solution is from 7.0 to. 8.0.

The present invention has the advantage that it provides hydrocortisone sodium phosphate aqueous pharmaceutical solutions suitable for oral administration more effectively preserved than the corresponding solutions of the prior art, and which may be stored in multi-dose containers, due to the inhibition of the growth of microorganisms caused by repeatedly withdrawing doses.

The present invention has also the advantage that it provides hydrocortisone sodium phosphate oral pharmaceutical solutions more physicochemically stable than the corresponding solutions of the prior art, by inhibiting oxidation that might occur, from repeatedly withdrawing doses thus allowing in this way their storage in multi-dose containers.

### DETAILED DESCRIPTION OF THE INVENTION

The present invention provides pharmaceutical solutions suitable for oral administration comprising hydrocortisone sodium phosphate as active ingredient in association with a pharmaceutically acceptable aqueous carrier.

As used throughout the present description and claims, the term «mg/ml», when referring to the active ingredient or inactive ingredients (excipients), means the milligrams of active or inactive ingredient per ml of oral solution.

As used throughout the present description and claims, the term "total impurities" refers to the sum of all hydrocortisone sodium phosphate impurities present in the oral solution, except for hydrocortisone.

As used throughout the present description and claims, the concentrations of sorbitol in the aqueous solutions of hydrocortisone sodium phosphate correspond to the concentration of anhydrous sorbitol.

Shelf life after first use (In-use shelf life), as established by the document of European Medicines Agency "Note for Guidance on the in-use stability testing of human medicinal products" (CPMPQWP/2934/99), is a period of time during which a multi-dose product can be used whilst retaining quality within an accepted specification once the container is first opened.

The ability of antimicrobial preservatives to inhibit the growth of, or kill, microorganisms in pharmaceutical preparations is evaluated through Antimicrobial Efficacy Tests (AETs). As used throughout the present description, the term "AET" refers to the test for efficacy of antimicrobial preservation described in paragraph 5.1.3 of the European Pharmacopoeia (EP). The test consists of challenging the preparation, with a prescribed inoculum of suitable microorganisms, storing the inoculated preparation at ambient temperature, avoiding sunlight, withdrawing samples from the container at specified intervals of time and counting the microorganisms in the samples so removed. The preservative properties of the preparation are adequate if, under the conditions of the test, there is a significant fall or no increase, as appropriate, in the number of microorganisms in the inoculated preparation after 2, 7, 14 and 28 days. The EP criteria for evaluation of antimicrobial activity in oral preparations are given in the following table in terms of decrease in the number of viable microorganisms against the value obtained for the inoculum.

**Table 1: EP, Criteria, for AET (limits in log reduction units)**

| | 2 days | 7 days | 14 days | 28 days |
|---|---|---|---|---|
| Bacteria | - | - | 3 | No increase |
| Moulds & Yeasts | - | - | 1 | No increase |

During pre-formulation studies, hydrocortisone sodium phosphate stability was investigated within a range of pH from 3.0 to 8.0. It was confirmed that a pH range from about 7.0 to about 8.0 is optimal for drug substance stability, i.e., from assay as well as hydrocortisone and total impurities content.

During development of oral hydrocortisone sodium phosphate solutions, it was also found that a preserved system is not easily achievable. Thus, the effectiveness of several preservatives in preventing the growth of microorganisms in hydrocortisone sodium phosphate solutions comprising minimum quantities of natural sweetening agents, and flavouring agents for alleviating the bitter after taste of hydrocortisone sodium phosphate was experimentally assessed.

Various concentrations of the following substances were employed in this study, i.e. sodium benzoate, benzoic acid, methylparaben, ethylparaben, butylparaben a combination of methylparaben and propylparaben, ethanol, sorbic acid, sodium bisulfite and propylene glycol. The effectiveness of these substances in preventing the growth of microorganisms in hydrocortisone sodium phosphate solutions, buffered at pH = 7.5, was determined. In these solutions, the concentration of hydrocortisone sodium phosphate was 5.37 mg/ml equivalent to 4 mg/ml hydrocortisone. All solutions contained 0.5 mg/ml orange flavour and 1.0 mg/ml sucralose.

The solutions were filled in multi-dose glass bottles closed with plastic screw caps and then studied for three months in relation to their stability after first use at storage conditions of 20 - 25°C. The three-month test simulated the common use of the product, since the containers were opened at least once a day and a certain amount of solution was withdrawn each time. The amount of solution withdrawn was selected so that enough solution remains in the containers to perform all analytical tests at the initial and final time points.

**Table 2: Test formulations and results**

| **Selected Preservative** | **Concentration (% w/v)** | **AET (T=3)** | **Appearance of solution (T=0)** | **Appearance of solution (T=3 months)** | **Total impurities (T=3 months)** |
|---|---|---|---|---|---|
| Sodium benzoate | 0.5 | Fail | Clear | White precipitate | Not performed |
| Benzoic acid | 0.5 | Fail | Clear | Clear | 4.1% |
| Methylparaben | 0.18 | Fail | Clear | Clear | 6.6% |
| Ethylparaben | 0.05 | Fail | Clear | White precipitate | Not performed |
| Butylparaben | 0.05 | Fail | Clear | Clear | 6.4% |
| Methylparaben | 0.12 | Fail | Clear | White precipitate | Not performed |
| and | | | | | |
| Propylparaben | 0.02 | | | | |
| Propylene glycol | 30 | Fail | Clear | Clear | 4.1% |
| Sorbic acid | 0.15 | Fail | Clear | Clear | 4.4% |
| and | | | | | |
| Propylene glycol | 30 | | | | |
| Ethanol | 20 | Fail | White precipitate | White precipitate | Not performed |
| Sorbic acid | 0.2 | Fail | Clear | Clear | 3.9% |
| Sodium bisulfite | 0.2 | Fail | Clear | White precipitate | Not performed |
| Sorbic bisulfite | 0.15 | Pass | Clear | Clear | 5.0% |
| and | | | | | |
| Propylene glycol | 30 | | | | |
| Methylparaben | 0.12 | Fail | Clear | White precipitate | Not performed |
| and | | | | | |
| Propylene glycol | 20 | | | | |

Indeed, it was found that not all preservatives provided sufficient preservation at the optimal pH of 7.5 especially against Staphylococcus aureus, Candida albicans and Aspergillus brasiliensis. Furthermore all solutions tested, proved to form a precipitate and/or exhibit high levels of impurities especially after repeated opening of the container indicating that hydrocortisone sodium phosphate is extensively degraded when the solution comes into direct contact with atmospheric conditions.

Under the above described circumstances the present inventors conducted an extensive research program with view towards solving the stability problems and low preservative effectiveness within the optimal for hydrocortisone sodium phosphate stability pH range of about 7.0 to 8.0. The present inventors developed oral solutions, which remain physicochemically stable and at the same time remain effectively preserved when stored for at least three months after first use at room temperature (20-25°C).
It has been surprisingly found that the synergistic use of propylene glycol and sorbitol at certain concentration ranges along with the adjustment of pH within the range 7.0 to 8.0, leads to stable hydrocortisone sodium phosphate aqueous compositions. Importantly, said compositions do not need to comprise additional excipients such as ethanol, parabens, sodium bisulfite and sodium formaldehyde sulfoxylate or other stabilizing excipients, which may raise additional safety and toxicity issues.

According to the invention, the oral pharmaceutical solutions comprise hydrocortisone sodium phosphate as active ingredient, 100 mg/ml to 200 mg/ml propylene glycol, 150 mg/ml to 250 mg/ml sorbitol, a pharmaceutically acceptable buffering agent and purified water wherein the pH of the oral solution is from 7.0 to 8.0.

Preferably the oral pharmaceutical solutions according to the invention comprise hydrocortisone sodium phosphate as active ingredient in concentrations equivalent to 2 mg/ml to 100 mg/ml hydrocortisone.

Preferably the oral pharmaceutical solutions according to the invention comprise purified water in concentrations greater than 500 mg/ml.

Any suitable buffering agent which acts as a buffer, in the basic pH range, can be used in the compositions of the present invention. Such buffering agents may include phosphate agents such as sodium and potassium monobasic phosphate, sodium, potassium and calcium citrate, as well as other pharmaceutically acceptable buffering agents. Preferably the pharmaceutically acceptable buffering agent is disodium dihydrogen phosphate dihydrate.

Suitable buffering agents work effectively if they lead to solutions having sufficient buffering capacity to resist the change in pH expected during production or storage period. Buffering agents when used in the compositions of the present invention should be added in quantities that lead to at least a minimum buffering capacity, so as to achieve pH values of the finished oral solution in the range of from 7.0 to 8.0.

Sorbitol may be added in the oral pharmaceutical solutions according to the invention in the form of sorbitol crystalline powder or crystallizing sorbitol liquid or partially dehydrated crystallizing sorbitol liquid or non-crystallizing sorbitol liquid as they are is described in the corresponding Ph. Eur. Monographs. Preferably, sorbitol is non-crystallizing sorbitol liquid, (i.e. sorbitol aqueous solution 70% w/w/) as described in the corresponding Ph. Eur. monograph.

The compositions of the present invention exhibit excellent stability even when they do not contain additional stabilizing agents. Thus, preferably the aqueous pharmaceutical solutions of hydrocortisone sodium phosphate of the present invention do not contain additional stabilizing agents, beyond propylene glycol and sorbitol, that may raise additional safety and toxicity issues. The absence of additional excipients that stabilize oral liquid compositions, such as ethanol, parabens, sodium bisulfite and sodium formaldehyde sulfoxylate is very important, particularly in the case of formulations intended for children.

The oral aqueous pharmaceutical solutions of hydrocortisone sodium phosphate, according to the invention may also optionally contain additional excipients commonly used in preparing oral liquid compositions, such as antioxidants, viscosity adjusting agents, natural sweetening agents, non-sugar based artificial sweetening agents and flavouring agents.

The antioxidants which may be used in the present invention comprise, amongst others, butylated hydroxytoluene, butylated hydroxyanisole, ethylenediamine tetraacetic acid ("EDTA"), ascorbic acid propyl gallate or any combination thereof.

The viscosity adjusting agents may be, for example, sodium carboxymethylcellulose, methylcellulose, hydroxypropylcellulose, polyvinylpyrrolidone, maltitol or any combination thereof.

The natural sweetening agents that may be used in the present invention comprise, amongst others, erythritol, xylitol, mannitol, maltitol, fructose, glucose, sucrose, maltose, or any combination thereof.
The non-sugar based artificial sweetening agents which may be used in the present invention comprise, amongst others, saccharin sodium, sucralose, saccharin, aspartame, acesulfame potassium or any combination thereof.

Appropriate flavouring agents which may be used in the present invention comprise, amongst others, any of the many non-toxic natural or artificial flavouring agents known in the daily practice. In particular, the flavouring agents used may include one or more of a variety of natural or artificial fruit flavours. Alternatively, or in addition, the flavouring agents may include one or more of natural or artificial vanilla, chocolate, and caramel flavourings, amongst others.

Hydrocortisone sodium phosphate oral aqueous pharmaceutical solutions of the present invention exhibit excellent in-use shelf life (i.e. shelf life after first use) when supplied in multi-dose containers. Specifically, they remain physicochemically stable and at the same time effectively preserved when stored at room temperature (20°C - 25°C) even when the containers are opened at least once a day for at least three months.

The compositions of the present invention may be prepared using methods well-known in the prior art. For example they may be prepared using the following process:
Half of the specified amount of purified water is added to the main vessel. The active ingredient (hydrocortisone sodium phosphate) is placed into the main vessel under continuous stirring until it is completely dissolved. The quantity of pH adjustment agent is added to the main vessel and dissolved. Sorbitol and propylene glycol are sequentially added to the above solution under continuous stirring until they are completely dissolved. The viscosity adjusting agent, the antioxidant, the sweetening agent and the flavouring agent, if present, are added to the above solution under continuous stirring until they are completely dissolved. The pH of the solution is adjusted to the desired value, if needed, using hydrochloric acid or sodium hydroxide solution. The volume of the solution is adjusted to the desired batch volume by adding purified water.

### EXAMPLES

The following examples show the influence of the proposed carrier, according to the invention, on the stability of hydrocortisone sodium phosphate and on the preservation of the oral solutions.

The solutions in examples 1 and 2 were prepared through a manufacturing process, where half of the specified amount of purified water was added to the main vessel. Hydrocortisone sodium phosphate was placed into the main vessel under continuous stirring until it was completely dissolved. The specified quantity of the pH adjustment agent (i.e. disodium dihydrogen phosphate dihydrate) was added to the main vessel and dissolved. Non-crystallizing sorbitol liquid and/or propylene glycol and/or glycerol were sequentially added to the above solution under continuous stirring until they are completely dissolved. The sweetening agent and the flavouring agent were added to the above solution under continuous stirring until they were completely dissolved. The pH of the solution was adjusted to the desired value using hydrochloric acid solution 0.1 N. The volume of the solution was adjusted to the desired batch volume by adding purified water. The solutions were transferred to multi-dose glass bottles that were then sealed with plastic screw caps.

### EXAMPLE 1

The compositions of this example do not belong to the present invention.

The prepared solutions were studied for three months in relation to their stability after first use in storage conditions of 20 - 25°C. The three-month test simulated the normal use of the product, since the containers were opened at least once a day and a certain amount of solution was withdrawn each time. The amount of solution withdrawn was selected so that enough solution remains in the containers to perform all analytical tests at the initial and final time points.

Quantification of hydrocortisone, hydrocortisone sodium phosphate and total impurities in the solutions was performed by HPLC.

The ability of the prepared solutions to inhibit the growth of, or kill, microorganisms after three months of repeated openings and closings of the container was evaluated through AETs carried out as described in paragraph 5.1.3 of the European Pharmacopoeia (EP).

**Table 3: Compositions A1 to A4**

| | **Composition** | | | |
|---|---|---|---|---|
| | **A1** | **A2** | **A3** | **A4** |
| **Active ingredient** | mg/ml | | | |
| Hydrocortisone Sodium Phosphate equivalent to | 5.37 | 5.37 | 5.37 | 5.37 |
| Hydrocortisone | 4 | 4 | 4 | 4 |

| **Excipients** | | | | |
|---|---|---|---|---|
| Propylene glycol | 100 | - | 200 | - |
| Sorbitol | 70 | 140 | - | - |
| Glycerol | 150 | 200 | 200 | 400 |
| Disodium dihydrogen phosphate dihydrate | 5.07 | 5.07 | 5.07 | 5.07 |
| Sucralose | 1.0 | 1.0 | 1.0 | 1.0 |
| Orange flavour | 0.5 | 0.5 | 0.5 | 0.5 |
| Purified water | q.s. 1ml | q.s. 1ml | q.s. 1ml | q.s. 1ml |
| HCl sol. 0.1 N | 7.5 | | | |
| **Assay (T= 0)** | 99.6% | 98.9% | 99.5% | 100.3% |
| **Assay (T= 3 months)** | 96.3% | 95.4% | 96.0% | 96.4% |
| **Hydrocortisone (T= 0)** | 0.07% | 0.09% | 0.06% | 0.11% |
| **Hydrocortisone (T= 3 months)** | 1.1% | 1.3% | 1.8% | 1.0% |
| **Total impurities (T= 0)** | 0.4% | 0.8% | 0.6% | 0.6% |
| **Total impurities (T= 3 months)** | 2.9% | 2.8% | 2.6% | 3.3% |
| **AET (T=3 months)** | Fail | Fail | Fail | Fail |

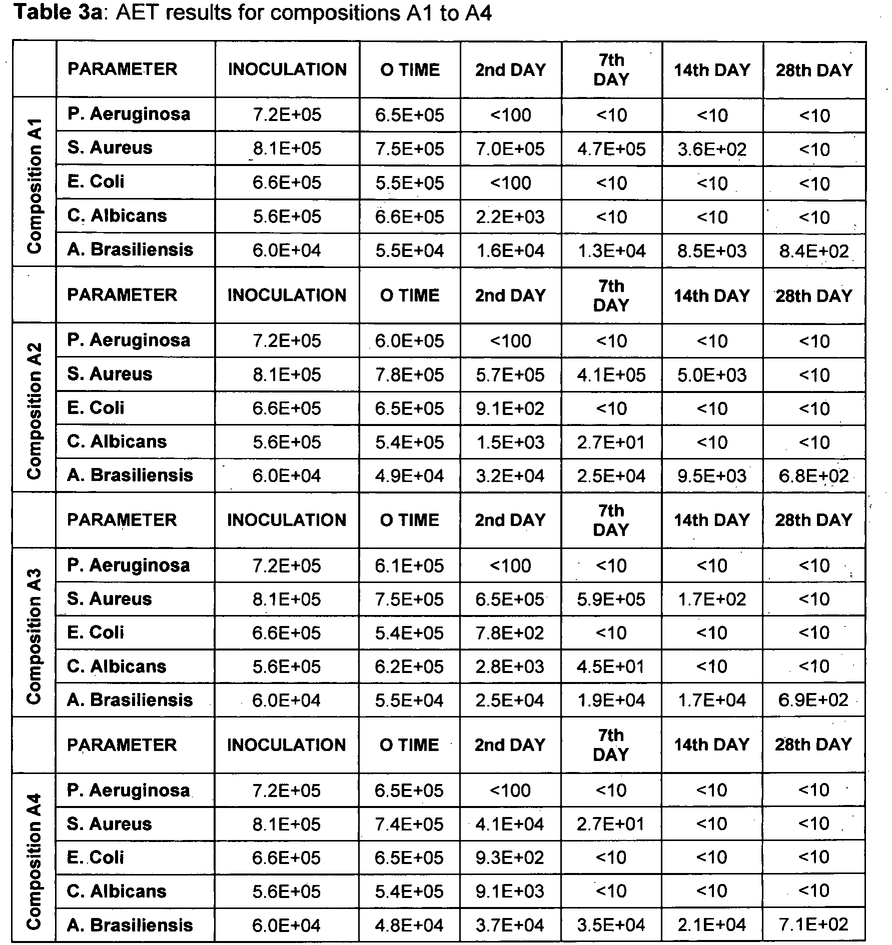

All solutions tested, proved to exhibit relatively high levels of impurities after repeated opening of the container. Furthermore, none of the aqueous carriers was found to provide sufficient preservation at the optimal pH of 7.5 especially against Staphylococcus aureus, Candida albicans and Aspergillus brasiliensis.

### EXAMPLE 2

This example demonstrates the effects of different concentrations of propylene glycol, sorbitol and hydrocortisone sodium phosphate on the stability and preservation of the solutions.

The prepared solutions were studied for three months in relation to their stability after first use in storage conditions of 20 - 25°C. Quantification of hydrocortisone, hydrocortisone sodium phosphate and total impurities in the solutions was performed by HPLC.

The ability of the prepared solutions to inhibit the growth of, or kill, microorganisms after three months of repeated openings and closings of the container was evaluated through AETs carried out as described in paragraph 5.1.3 of the European Pharmacopoeia (EP).

**Table 4: Compositions with increasing sorbitol concentration (B1 to B5)**

| | **Composition** | | | | |
|---|---|---|---|---|---|
| | **B1** | **B2** | **B3** | **B4** | **B5** |
| **Active ingredient** | mg/ml | | | | |
| Hydrocortisone Sodium Phosphate equivalent to | 5.37 | 5.37 | 5.37 | 5.37 | 5.37 |
| Hydrocortisone | 4 | 4 | 4 | 4 | 4 |

| **Excipients** | | | | | |
|---|---|---|---|---|---|
| Propylene glycol | 170 | 170 | 170 | 170 | 170 |
| Sorbitol | 35 | 49 | 140 | 175 | 210 |
| Disodium dihydrogen phosphate dihydrate | 5.07 | 5.07 | 5.07 | 5.07 | 5.07 |
| Purified water | q.s. 1ml | q.s. 1ml | q.s. 1ml | q.s. 1 ml | q.s. 1ml |
| HCl sol. 0.1 N | q.s. pH = 7.5 | | | | |
| **Assay (T=** 0) | 99.4% | 100.0% | 99.8% | 99.6% | 100.4% |
| **Assay (T= 3 months)** | 95.6% | 97.5% | 98.9% | 98.7% | 99.1% |
| **Hydrocortisone (T= 0)** | 0.07% | 0.1% | 0.06% | 0.04% | 0.09% |
| **Hydrocortisone (T= 3 months)** | 1.6% | 0.8% | 0.4% | 0.6% | 0.5% |
| **Total impurities (T= 0)** | 0.6% | 0.9% | 0.4% | 0.5% | 0.6% |
| **Total impurities (T= 3 months)** | 3.3% | 3.0% | 0.8% | 1.0% | 1.8% |
| **AET (T=3 months)** | Fail | Pass | Pass | Pass | Fail |

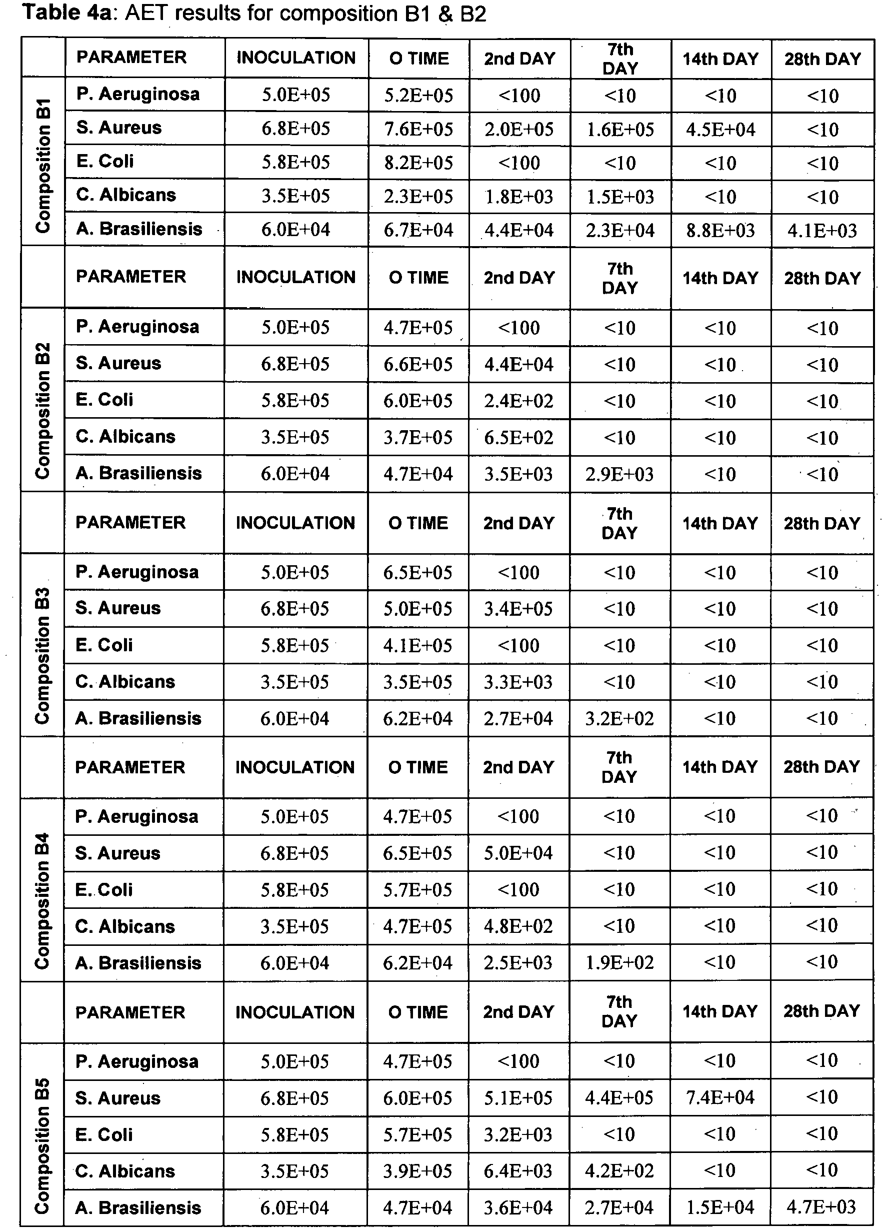

Although propylene glycol does have some inhibitory effect on the growth of the tested bacteria and fungi in solutions of hydrocortisone sodium phosphate, it is not effective on its own against the growth of certain organisms. It is revealed that only a combination of propylene glycol and sorbitol, at certain concentration ranges, is generally effective in preserving and at the same time physicochemically stabilizing aqueous hydrocortisone sodium phosphate solutions.

**Table 5: Compositions with increasing propylene glycol concentrations (C1 to C5)**

| | **Composition** | | | | |
|---|---|---|---|---|---|
| | **C1** | **C2** | **C3** | **C4** | **C5** |
| **Active ingredient** | mg/ml | | | | |
| Hydrocortisone Sodium Phosphate equivalent to | 5.37 | 5.37 | 5.37 | 5.37 | 5.37 |
| Hydrocortisone | 4 | 4 | 4 | 4 | 4 |

| **Excipients** | | | | | |
|---|---|---|---|---|---|
| Propylene glycol | 50 | 70 | 150 | 200 | 300 |
| Sorbitol | 119 | 119 | 119 | 119 | 119 |
| Disodium dihydrogen phosphate dihydrate | 5.07 | 5.07 | 5.07 | 5.07 | 5.07 |
| Purified water | q.s. 1 ml | q.s. 1ml | q.s. 1ml | q.s. 1ml | q.s. 1ml |
| HCl sol. 0.1 N | q.s. pH = 7.5 | | | | |
| **Assay (T=** 0) | 99.8% | 99.6% | 99.8% | 99.6% | 100.4% |
| **Assay (T= 3 months)** | 96.3% | 97.5% | 98.4% | 98.7% | 98.0% |
| **Hydrocortisone (T= 0)** | 0.06% | 0.04% | 0.06% | 0.04% | 0.09% |
| **Hydrocortisone (T= 3 months)** | 0.4% | 0.6% | 0.4% | 0.6% | 0.7% |
| **Total impurities (T= 0)** | 0.4% | 0.5% | 0.4% | 0.5% | 0.3% |
| **Total impurities (T= 3 months)** | 3.3% | 2.2% | 1.3% | 1.0% | 2.5% |
| **AET (T=3 months)** | Fail | Fail | Pass | Pass | Fail |

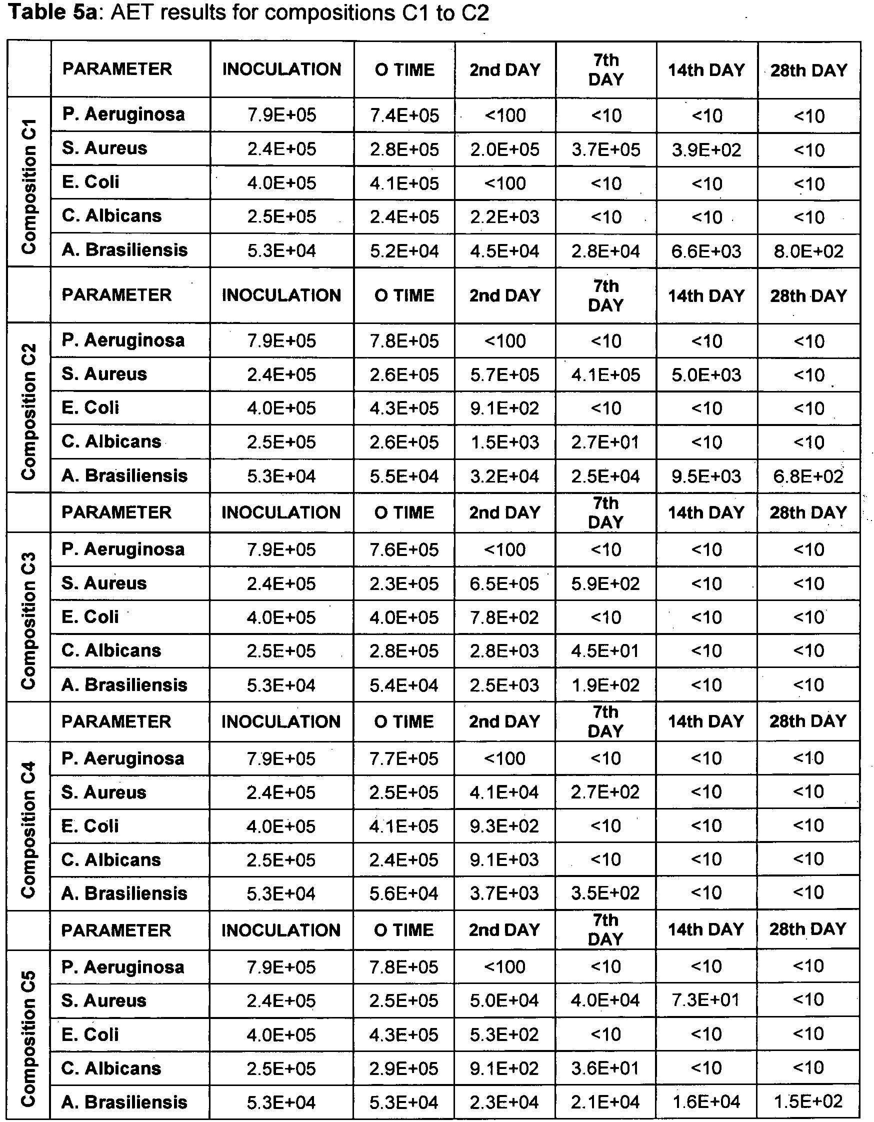

**Table 6: Compositions with increasing concentration of the active ingredient**

| | **Composition** | | | |
|---|---|---|---|---|
| | **D1** | **D2** | **D3** | **D4** |
| **Active ingredient** | mg/ml | | | |
| Hydrocortisone Sodium Phosphate equivalent to | 8.04 | 10.72 | 13.4 | 134.0 |
| Hydrocortisone | 6 | 8 | 10 | 100 |

| **Excipients** | mg/ml | | | |
|---|---|---|---|---|
| Sorbitol | 140 | 140 | 140 | 140 |
| Propylene glycol | 200 | 200 | 200 | 200 |
| Disodium dihydrogen phosphate dihydrate | 5.07 | 5.07 | 5.07 | 5.07 |
| Purified water | q.s. 1ml | q.s. 1ml | q.s. 1ml | q.s. 1ml |
| HCl sol. 0.1 N | q.s. pH = 7.5 | | | |
| **Assay (T=** 0) | 101.3% | 100.9% | 100.4% | 100.6% |
| **Assay (T= 3 months)** | 99.9% | 99.3% | 99.1% | 98.8% |
| **Hydrocortisone (T= 0)** | 0.09% | 0.15% | 0.13% | 0.16% |
| **Hydrocortisone (T= 3 months)** | 0.7% | 0.7% | 0.8% | 1.0% |
| **Total impurities (T= 0)** | 0.4% | 0.3% | 0.4% | 0.5% |
| **Total impurities (T= 3 months)** | 0.9% | 1.0% | 1.1% | 1:4% |
| **AET (T=3 months)** | Pass | Pass | Pass | Pass |

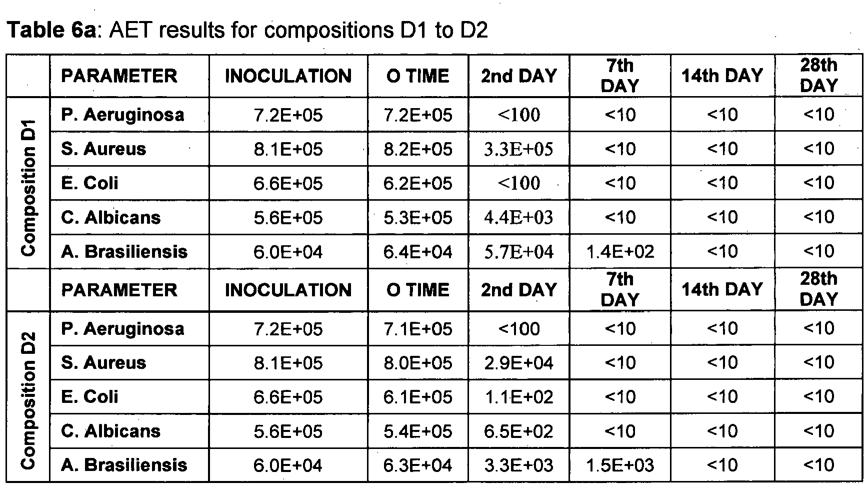

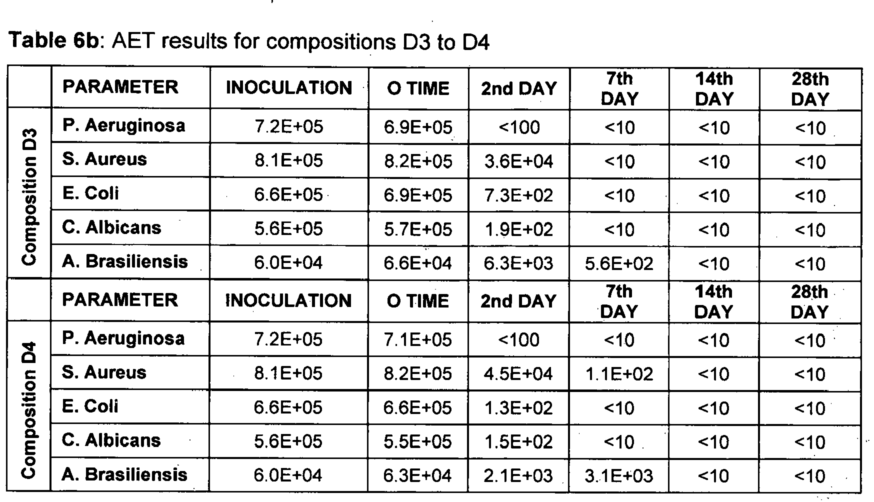

It was found that the proposed aqueous carrier provides stable compositions independent of the concentration of hydrocortisone sodium phosphate.

### EXAMPLE 3

Table 7 shows preferred compositions according to the present invention.

The following solutions were prepared through a manufacturing process, where half of the specified amount of purified water was added to the main vessel. Hydrocortisone sodium phosphate was placed into the main vessel under continuous stirring until it was completely dissolved. The quantity of disodium dihydrogen phosphate dihydrate was added to the main vessel and dissolved. Non-crystallizing sorbitol liquid and propylene glycol were sequentially added to the above solution under continuous stirring until they were completely dissolved. Disodium edetate, sucralose and orange flavour were added to the above solution under continuous stirring until they were completely dissolved. The pH of the solution was adjusted to the desired value using hydrochloric acid solution 0.1 N. The volume of the solution was adjusted to the desired batch volume by adding purified water.

**Table 7: Preferred compositions according to the present invention**

| | **Composition E1** | **Composition E2** |
|---|---|---|
| **Active ingredient** | mg/ml | |
| Hydrocortisone Sodium Phosphate equivalent to Hydrocortisone | 5.37 | 10.74 |
| | 4 | 8 |

| **Excipients** | | |
|---|---|---|
| Propylene glycol | 180.0 | 200.0 |
| Sorbitol | 140.0 | 126.0 |
| Disodium edetate | 1.0 | 1.0 |
| Disodium dihydrogen phosphate dihydrate | 5.07 | 5.07 |
| Sucralose | 1.0 | 1.0 |
| Orange flavour | 0.5 | 1.5 |
| Purified water | q.s. 1ml | q.s. 1ml |
| hydrochloric acid solution, 0.1 N | q.s. to pH: 7.5 | q.s. to pH: 7.5 |

Solutions of Table 7 were studied for three months with regard to their physicochemical stability and preservation effectiveness after first use in storage conditions of 20 - 25°C. Quantification of free hydrocortisone, hydrocortisone sodium phosphate and its impurities in the solutions was performed by HPLC.

**Table 7a: Stability after first use (in-use stability)**

| | **Composition E1** | **Composition E2** |
|---|---|---|
| **Assay (T= 0)** | 101.1% | 100.8% |
| **Assay (T= 3 months)** | 99.9% | 99.3% |
| **Hydrocortisone (T= 0)** | 0.4% | 0.6% |
| **Hydrocortisone (T= 3 months)** | 0.8% | 1.1% |
| **Total impurities (T= 0)** | 0.6% | 0.8% |
| **Total impurities (T= 3 months)** | 1.1% | 1.4% |
| **AET (T=3 months)** | Pass | Pass |

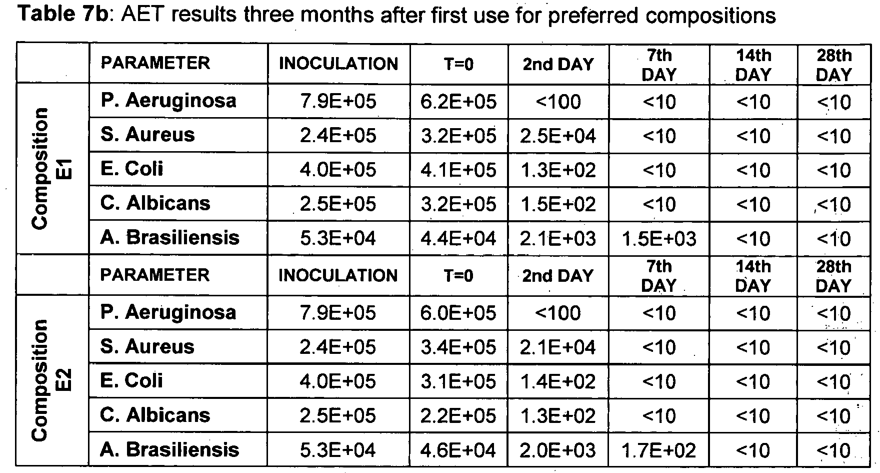

## Claims

1. Oral pharmaceutical solution comprising hydrocortisone sodium phosphate, from 100 mg/ml to 200 mg/ml propylene glycol, from 100 mg/ml to 200 mg/ml sorbitol, a pharmaceutically acceptable buffering agent and purified water, wherein the pH of the oral solution is from 7.0 to 8.0.

2. Oral pharmaceutical solution according to claim 1, wherein the concentration of hydrocortisone sodium phosphate is equivalent to from 2 mg/ml to 100 mg/ml hydrocortisone.

3. Oral pharmaceutical solution according to any one of claims 1 and 2, wherein the concentration of purified water is greater than 500 mg/ml.

4. Oral pharmaceutical solution according to any one of claims 1 To 3, wherein sorbitol is non-crystallizing liquid sorbitol.

5. Oral pharmaceutical solution according to any one of claims 1 to 4, wherein the buffering agent is disodium dihydrogen phosphate dihydrate.

6. Oral pharmaceutical solution according to any one of claims 1 to 5, which is free of additional stabilizing agents, beyond propylene glycol and sorbitol.

7. Oral pharmaceutical solution according to any one of claims 1 to 6, which is free of parabens, sorbic acid or sodium benzoate.

8. Oral pharmaceutical solution according to any one of claims 1 to 7, which is free of glycerol.

9. Oral pharmaceutical solution according to any one of claims 1 to 8, which is free of sulfites or bisulfites.

10. Oral pharmaceutical solution according to any one of claims 1 to 9, which consists of
5.37 mg/ml hydrocortisone sodium phosphate,
180.0 mg/ml propylene glycol,
140.0 mg/ml sorbitol,
1.0 mg/ml disodium edetate,
5:07 mg/ml disodium dihydrogen phosphate dihydrate,
1.0 mg/ml sucralose,
0.5 mg/ml orange flavour
and purified water, wherein the pH of the oral solution is 7.5.

11. Oral pharmaceutical solution according to any one of claims 1 to 9, which consists of
10.74 mg/ml hydrocortisone sodium phosphate,
200 mg/ml propylene glycol,
126 mg/ml sorbitol,
1.0 mg/ml disodium edetate,
5.07 mg/ml disodium dihydrogen phosphate dihydrate,
1.0 mg/ml sucralose,
1.5 mg/ml orange flavour
and purified water, wherein the pH of the oral solution is 7.5.

## Patentansprüche

1. Orale pharmazeutische Lösung, die Hydrocortison-Natriumphosphat, 100 mg/ml bis 200 mg/ml Propylenglycol, 100 mg/ml bis 200 mg/ml Sorbitol, ein pharmazeutisch akzeptables Puffermittel und gereinigtes Wasser enthält, wobei der pH-Wert der oralen Lösung 7,0 bis 8,0 ist.

2. Orale pharmazeutische Lösung nach Anspruch 1, wobei die Konzentration von Hydrocortison-Natriumphosphat äquivalent zu 2 mg/ml bis 100 mg/ml Hydrocortison ist.

3. Orale pharmazeutische Lösung nach einem der Ansprüche 1 und 2, wobei die Konzentration des gereinigten Wassers größer als 500 mg/ml ist.

4. Orale pharmazeutische Lösung nach einem der Ansprüche 1 bis 2, wobei Sorbitol nicht-kristallisierendes flüssiges Sorbitol ist.

5. Orale pharmazeutische Lösung nach einem der Ansprüche 1 bis 4, wobei das Puffermittel Dinatriumdihydrogenphosphatdihydrat ist.

6. Orale pharmazeutische Lösung nach einem der Ansprüche 1 bis 5, die frei von zusätzlichen Stabilisierungsmitteln ist, außer Propylen- Glykol und Sorbitol.

7. Orale pharmazeutische Lösung nach einem der Ansprüche 1 bis 6, die frei von Parabenen, Sorbinsäure oder Natriumbenzoat ist.

8. Orale pharmazeutische Lösung nach einem der Ansprüche 1 bis 7, die frei von Glycerol ist.

9. Orale pharmazeutische Lösung nach einem der Ansprüche 1 bis 8, die frei von Sulfiten oder Bisulfiten ist.

10. Orale pharmazeutische Lösung nach einem der Ansprüche 1 bis 9, die besteht aus
5,37 mg/ml Hydrocortison-Natriumphosphat,
180,0 mg/ml Propylenglycol,
140,0 mg/ml Sorbitol,
1,0 mg/ml Dinatriumedetat,
5,07 mg/ml Dinatriumdihydrogenphosphatdihydrat,
1,0 mg/ml Sucralose,
0,5 mg/ml Orangenaroma
und gereinigtem Wasser, wobei der pH-Wert der oralen Lösung 7,5 beträgt.

11. Orale pharmazeutische Lösung nach einem der Ansprüche 1 bis 9, die besteht aus
10,74 mg/ml Hydrocortison-Natriumphosphat,
200 mg/ml Propylenglycol,
126 mg/ml Sorbitol,
1,0 mg/ml Dinatriumedetat,
5,07 mg/ml Dinatriumdihydrogenphosphatdihydrat,
1,0 mg/ml Sucralose,
1,5 mg/ml Orangenaroma
und gereinigtem Wasser, wobei der pH-Wert der oralen Lösung 7,5 beträgt.

## Revendications

1. Solution pharmaceutique orale comprenant du phosphate sodique d'hydrocortisone, de 100 mg/ml à 200 mg/ml de propylène glycol, de 100 mg/ml à 200 mg/ml de sorbitol, un agent tampon pharmaceutiquement acceptable et de l'eau purifiée, dans laquelle le pH de la solution orale est compris entre 7,0 et 8,0.

2. Solution pharmaceutique orale selon la revendication 1, dans laquelle la concentration en phosphate sodique d'hydrocortisone est équivalente à 2 mg/ml à 100 mg/ml d'hydrocortisone.

3. Solution pharmaceutique orale selon l'une quelconque des revendications 1 et 2, dans laquelle la concentration en eau purifiée est supérieure à 500 mg/ml.

4. Solution pharmaceutique orale selon l'une quelconque des revendications 1 à 3, dans laquelle le sorbitol est du sorbitol liquide ne cristallisant pas.

5. Solution pharmaceutique orale selon l'une quelconque des revendications 1 à 4, dans laquelle l'agent tampon est le dihydrogénophosphate disodique dihydraté.

6. Solution pharmaceutique orale selon l'une quelconque des revendications 1 à 5, qui est dépourvue d'agents stabilisants supplémentaires, outre le propylène glycol et le sorbitol.

7. Solution pharmaceutique orale selon l'une quelconque des revendications 1 à 6, qui est dépourvue de parabènes, d'acide sorbique ou de benzoate de sodium.

8. Solution pharmaceutique orale selon l'une quelconque des revendications 1 à 7, qui est dépourvue de glycérol.

9. Solution pharmaceutique orale selon l'une quelconque des revendications 1 à 8, qui est dépourvue de sulfites ou de bisulfites.

10. Solution pharmaceutique orale selon l'une quelconque des revendications 1 à 9, qui est constituée de :
5,37 mg/ml de phosphate sodique d'hydrocortisone,
180,0 mg/ml de propylène glycol,
140,0 mg/ml de sorbitol,
1,0 mg/ml d'édétate disodique,
5,07 mg/ml de dihydrogénophosphate disodique dihydraté,
1,0 mg/ml de sucralose,
0,5 mg/ml d'arôme orange,
et de l'eau purifiée, dans laquelle le pH de la solution orale est de 7,5.

11. Solution pharmaceutique orale selon l'une quelconque des revendications 1 à 9, qui est constituée de :
10,74 mg/ml de phosphate sodique d'hydrocortisone,
200 mg/ml de propylène glycol,
126 mg/ml de sorbitol,
1,0 mg/ml d'édétate disodique,
5,07 mg/ml de dihydrogénophosphate disodique dihydraté,
1,0 mg/ml de sucralose,
1,5 mg/ml d'arôme orange,
et de l'eau purifiée, dans laquelle le pH de la solution orale est de 7,5.
